# FASCICULE DE BREVET EUROPEEN

(11) **EP 0 833 895 B1**
(45) Date de publication et mention de la délivrance du brevet: **09.11.2005**
(21) Numéro de dépôt: 97920791.7
(22) Date de dépôt: 18.04.1997
(51) Int. Cl.: C12N 5/10

(54) **LIGNEES IMMORTALISEES DE CELLULES RETINIENNES ET LEURS APPLICATIONS**
UNSTERBLICHE RETINALZELLINIEN UND DEREN ANWENDUNGEN
IMMORTALIZED RETINAL CELL LINES AND THEIR APPLICATION

(30) Priorité: 19.04.1996 FR 9604964
(43) Date de publication de la demande: 08.04.1998
(73) Titulaire: Neurotech S.A., 91000 Evry (FR)
(72) Inventeur: GREENWOOD, John, London SE5 8BN (GB); ADAMSON, Peter, Croydon CR0 7RZ (GB); LUND, Raymond, London EC1Y 8QA (GB)
(74) Mandataire: Breese, Pierre
(86) Numéro de dépôt international: PCT/FR1997/000709
(87) Numéro de publication internationale: WO 1997/040139

(56) Documents cités:
- NEURON, vol. 4, no. 5, Mai 1990, pages 775-782, XP000615818 JOSEPH P. HAMMANG ET AL.: "IMMORTALIZED RETINAL NEURONS DERIVED FROM SV40 T-ANTIGEN-INDUCED TUMORS IN TRANSGENIC MICE."
- ONCOGENE, vol. 5, no. 2, Février 1990, pages 195-200, XP000615816 K. DUTT ET AL.: "ESTABLISHMENT OF HUMAN RETINAL PIGMENT EPITHELIAL CELL LINES BY ONCOGENES."
- DNA AND CELL BIOLOGY, vol. 13, no. 9, Septembre 1994, pages 909-921, XP000615992 KAMLA DUTT ET AL.: "ESTABLISHMENT OF A HUMAN RETINAL CELL LINE BY TRANSFECTION OF SV40 T ANTIGEN GENE WITH POTENTIAL TO UNDERGO NEURONAL DIFFERENTIATION."
- CURRENT EYE RESEARCH, vol. 15, no. 5, Mai 1996, pages 477-485, XP000615815 DUTT K ET AL: "PROTO-ONCOGENE EXPRESSION IN CAMP AND TPA-MEDIATED NEURONAL DIFFERENTIATION IN A HUMAN RETINAL CELL LINE KGLDMSM"

## Description

La présente invention est relative à de nouvelles lignées cellulaires immortalisées, d'origine rétinienne (épithéliale pigmentaire rétinienne) aptes à être implantées dans la rétine et aptes à véhiculer une substance d'intérêt thérapeutique au niveau de l'oeil et du système nerveux central. De telles lignées peuvent également servir de modèle d'étude des interfaces sang-système nerveux central.

Aussi bien la barrière hémato-encéphalique que la barrière hémato-rétinienne sont importantes dans le contrôle du passage de substances vers et à partir du parenchyme neural, notamment dans le maintien de l'hémostase.

Dans la rétine, la barrière hémato-rétinienne comprend deux types cellulaires distincts, qui sont séparés anatomiquement. L'endothélium vasculaire rétinien, qui alimente la portion antérieure de la rétine, est actuellement considéré comme étant de structure identique à celle de l'endothélium cérébral (Towler et al., J. Physiol., 1994, **480,** 10-11P), alors que les cellules de l'épithélium pigmentaire rétinien recouvrent les vaisseaux perméables de la circulation choroïdienne et forment la barrière postérieure au moyen de leurs jonctions apicales serrées et sont en conséquence similaires aux cellules épithéliales à jonctions serrées des plexus choroïdes.

Les endothéliums cérébral et rétinien sont de nature différente de l'endothélium périphérique et n'ont pas pour uniques fonctions d'exprimer des jonctions serrées et de former une barrière physique. D'autres propriétés de l'endothélium contribuent à la nature spécialisée de cette barrière ; il s'agit en particulier de la distribution et de l'expression de substances telles que le transporteur du glucose (GLUT-1), le récepteur de la transferrine et le produit d'expression du gène de résistance aux médicaments, la P-glycoprotéine (Pgp).

Les endothéliums cérébral et rétinien diffèrent également de l'endothélium périphérique, par leur perméabilité aux leucocytes circulants.

L'étude *in vitro* des mécanismes moléculaires de l'induction du phénotype endothélial présente l'inconvénient d'être dépendante de la disponibilité en cultures cellulaires primaires de cellules endothéliales de vaisseaux cérébraux ou rétiniens ou de cellules épithéliales pigmentaires de la rétine.

Pour ce qui concerne le transfert *in vivo,* l'utilisation de tissus nerveux primaires d'origine foetale, pour la transplantation cellulaire, en thérapie humaine est une source de nombreux problèmes éthiques et pratiques ; une alternative à ce problème est l'utilisation de lignées cellulaires primaires d'origine neurale (neurones, cellules gliales, astrocytes par exemple) ou de lignées cellulaires non neurales (fibroblastes, myoblastes, cellules chromaffines de la médullo-surrénale, hépatocytes par exemple). Bien que les lignées cellulaires de médullo-surrénale, de fibroblastes ou de myoblastes puissent effectivement libérer des substances actives *in vivo,* elles ne sont pas normalement présentes dans le système nerveux central, peuvent modifier la fonction normale du système nerveux et entraîner une réaction de rejet. Nabi et al., (Journal of Cell Science, 104, 37-49 (1993)), décrit une lignée de cellules pigmentaires de rétine de rat immortalisée.

En raison de l'hétérogénéité des cellules endothéliales des différents tissus, influencée par l'environnement de ces cellules, il était important de pouvoir disposer de cellules adaptées à l'environnement rétinien, pour disposer d'outils permettant une bonne intégration morphologique et physiologique des cellules, lorsqu'elles sont implantées ou greffées.

En conséquence, les Inventeurs se sont donnés pour but de pourvoir à des lignées de cellules immortalisées, dérivées de cultures primaires de de l'épithélium pigmentaire rétinien de mammifère, notamment de rongeurs et plus particulièrement de rats, qui répondent mieux au besoin de la pratique, notamment en ce que toutes les lignées obtenues sont stables et présentent la plupart des caractéristiques des cellules différenciées.

La présente invention a pour objet des lignées immortalisées de cellules de mammifères, caractérisées en ce qu'elles sont dérivées de cultures primaires rétiniennes, sélectionnées dans le groupe constitué par les cellules primaires épithéliales de rétine, en ce qu'elles comprennent un fragment d'acide nucléique comprenant au moins un fragment immortalisant d'un oncogène viral thermo-sensible, lequel fragment d'acide nucléique est éventuellement associé à au moins un gène de sélection et en ce qu'elles présentent, de manière stable, les caractéristiques morphologiques et au moins les caractéristiques d'expression des antigènes de surface des cellules de cultures primaires correspondantes.

On entend par stabilité, le maintien des caractéristiques morphologiques et phénotypiques des lignées immortalisées au moins jusqu'à 30 passages et même jusqu'à plus de 50 passages.

Selon un mode de réalisation avantageux desdites lignées, elles sont dérivées de cellules épithéliales pigmentaires de rétine et présentent les caractéristiques morphologique et d'expression antigénique des cellules épithéliales pigmentaire rétiniennes de culture primaire : morphologie pavimenteuse, expression de RET-PE2 et des cytokératines.

De manière surprenante, de telles cellules ne diffèrent pas dans leur expression des caractéristiques des cellules épithéliales pigmentaires rétiniennes différenciées.

En outre :
. les cellules épithéliales pigmentaires rétiniennes sont capables, *in vivo*, de s'intégrer de manière appropriée à la cytoarchitecture de la rétine d'un mammifère hôte sans proliférer et de prévenir la perte des photorécepteurs, notamment chez le rat de souche RCS (*Royal College Surgeon*) et

Selon un autre mode de réalisation avantageux desdites lignées, le fragment d'acide nucléique comprenant au moins un fragment immortalisant d'un oncogène contient un fragment d'un oncogène grand T de SV40 thermo-sensible.

On a ainsi obtenu :
des cellules épithéliales pigmentaires rétiniennes immortalisées, dénommées IO/LD7/4, très similaires aux cellules de culture primaire ; bien que non pigmentées, ces cellules expriment l'antigène spécifique RET-PE2 et les cytokératines.

Conformément à l'invention, les cellules épithéliales pigmentaires rétiniennes immortalisées, dénommées IO/LD7/4 ont été déposées sous le n° I-1694 en date du 18 avril 1996 auprès de la Collection nationale de Cultures de Micro-organismes tenue par l'Institut Pasteur.

La présente invention a également pour objet des lignées cellulaires issues des lignées immortalisées telles que définies ci-dessus dénommées ci-après lignées de cellules vectrices, caractérisées en ce qu'elles comprennent au moins une lignée cellulaire telle que définie ci-dessus associée à un vecteur d'expression comprenant une séquence codant pour un polypeptide, une protéine ou un vecteur viral, éventuellement associé à au moins un gène de sélection et éventuellement au moins un gène marqueur et en ce qu'elles sont capables *in vivo* de s'intégrer à la rétine et notamment à l'espace sous-rétinien d'un mammifère hôte, à prévenir la perte des photorécepteurs et à produire ledit peptide, ladite protéine ou ledit vecteur viral.

On entend au sens de la présente invention,' par vecteur d'expression, tout fragment d'acide nucléique intégré dans le génome ou présent au niveau cytoplasmique desdites lignées cellulaires et capable de permettre l'expression desdits polypeptides, protéine ou vecteur viral.

L'ensemble des lignées selon la présente invention (lignées cellulaires et lignées vectrices) a l'avantage de constituer une source pure, homogène et suffisante de cellules d'origine rétinienne, en vue d'une application reproductible aux transplantations, notamment parce que l'ensemble de ces lignées présentent le phénotype des lignées de cultures primaires.

En ce qui concerne plus particulièrement les lignées vectrices, elles s'intègrent bien à la vascularisation rétinienne, sont très bien tolérées et libèrent *in vivo,* sur une longue période, la substance active qu'elles expriment et trouvent application dans la préparation d'une composition pour le traitement des troubles primaires ou secondaires ophtalmologiques ou neurologiques.

La présente invention a en outre pour objet un modèle d'étude et d'identification des systèmes biochimiques et cellulaires de la barrière hémato-rétinienne, caractérisé en ce qu'il comprend au moins une lignée cellulaire telle que définie ci-dessus.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre la morphologie des cultures primaires de cellules endothéliales rétiniennes (C), de cellules épithéliales pigmentaires rétiniennes (E) et des clones immortalisés IO/JG2/1 (D) et IO/LD7/4 (F) selon l'invention ;
- la figure 2 illustre en microscopie électronique à transmission, les cellules IO/LD7/4 (A) et IO/JG2/1 (C, E) ;
- la figure 3 illustre la coloration nucléaire obtenue en présence d'anticorps dirigés contre l'antigène grand T : IO/JG2/1 (B) et IO/LD7/4 (C) ;
- la figure 4 illustre l'expression de différents marqueurs endothéliaux dans les cultures IO/JG2/1 (A-F) ;
- la figure 5 illustre l'expression comparée de différents marqueurs épithéliaux dans les cultures primaires (A, B) et le clone IO/LD7/4 (C, D) ;
- la figure 6 illustre l'expression de la molécule d'adhésion ICAM-1 (A, B), des antigènes du système majeur d'histocompatibilité de classe I (C, D) et de classe II (E, F), dans le clone IO/LD7/4 en l'absence (A, C, E) ou en présence (B, D, F) d'induction par IFN-γ ;
- la figure 7 illustre, pour les cellules IO/LD7/4, l'expression des molécules d'adhésion ICAM-1 (B, barres noires) et VCAM-1 (B, barres hachurées) ainsi que des antigènes d'histocompatibilité de classe II I-A (A, noir) et I-E (A, hachuré) en réponse à l'IFN-γ de 0 à 5 jours ;
- la figure 8 illustre la migration des lymphocytes T à travers des monocouches constituées des cultures primaires de cellules endothéliales rétiniennes (REC), pigmentaires épithéliales rétiniennes (RPE) ou des clones IO/JG2/1 et IO/LD7/4 ;
- la figure 9 illustre des électromicrographies de cellules IO/LD7/4 cocultivées avec de la rétine dissociée ; les débris de segments externes (ROS) sont adjacents aux cellules et retrouvés dans les phagosomes (P) ;
- la figure 10 illustre des cellules IO/LD7/4 cultivées sur des lamelles recouvertes de Matrigel® : les cellules montrent une capacité contractile forte, créant des lignes de stress dans la matrice ;
- la figure 11 illustre la morphologie hexagonale des cellules obtenues après greffe des cellules IO/LD7/4 chez le rat Sprague-Dawley au niveau de la rétine ;
- la figure 12 montre les moyennes et les écart-types des temps de latence des réflexes pupillaires en réponse à un stimulus lumineux, des rats greffés avec des cellules RPE primaires (figure 12A), avec des cellules IO/LD7/4 (figure 12B) et des animaux contrôles (opération à blanc) (figure 12C) ; les données de la figure 12D montrent les réponses d'un rat RCS dystrophique en fonction de 1'âge ; le temps de latence moyen d'un rat non-dystrophique est de 0,48 ± 0,04 secondes ; L = oeil gauche et R = oeil droit ;
- la figure 13 illustre les moyennes et les écart-types de l'amplitude des réponses du réflexe pupillaire à la lumière des rats greffés avec des cellules RPE primaires (figure 13A), avec des cellules IO/LD7/4 (figure 13B) ou des rats contrôles (figure 13C) ; les données illustrées à la figure 13D correspondent aux réponses d'un rat RCS dystrophique en fonction de l'âge ; l'amplitude moyenne de réponse d'un animal non-dystrophique de 6 mois est de 19,7 ± 5,7 % ; L = oeil gauche et R = oeil droit ;
- la figure 14 illustre les modifications d'activité moyenne de rats placés dans des cages présentant des parois à motifs différents ; rats greffés avec des cellules IO/LD7/4 (barres hachurées), rats contrôle (opération à blanc « *sham* » ; barres *blanches); blanck =* parois unies ; *check =* parois décorées ;
- la figure 15 illustre le nombre d'unités actives de champ visuel dans le collicule supérieur, exprimé en pourcentage du nombre total d'enregistrements ; les cellules IO/LD7/4 et RPE primaires sont capables de ralentir la perte du champ visuel chez les animaux greffés, par rapport aux animaux non-greffés (*control* ou *sham*) ;

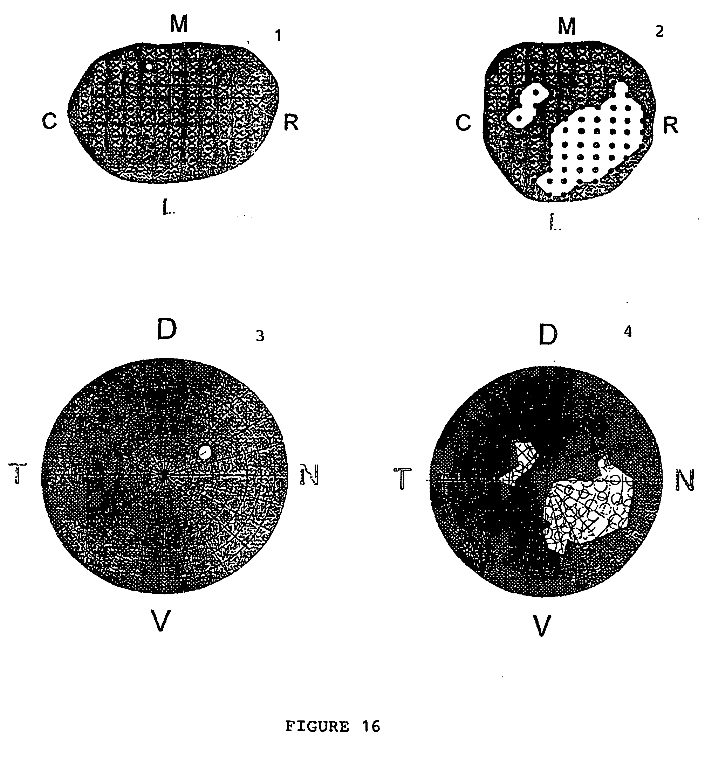

- la figure 16 représente d'une part, une vue en deux dimensions du collicule supérieur (figures 16.1 et 16.2, dans lesquelles C = caudal, M = médial, R = rostral et L = latéral) et d'autre part, les cartes des champs visuels correspondants de la rétine (figures 16.3 et 16.4, dans lesquelles D = dorsal, N = nasal, V = ventral et T = temporal) ; les croix sur la carte du collicule représentent les zones à partir desquelles aucun enregistrement n'a pu être obtenu ; les points correspondent aux zones pour lesquelles des enregistrements ont pu être obtenus ; les figures de gauche (figures 16.1 et 16.3) représentent les enregistrements d'un rat dystrophique de 6 mois : les enregistrements ont pu être réalisés à partir d'une seule unité (zone claire), ce qui est typique des animaux de cet âge. Les figures de droite (figures 16.2 et 16.4) représentent les enregistrements d'un rat, greffé avec des cellules IO/LD7/4, au niveau de la rétine temporale supérieure. On observe une large zone du collicule à partir de laquelle des réponses peuvent être obtenues ;
- la figure 17 illustre quelques unes des différences relatives aux caractéristiques histologiques (nombre de noyaux dans la couche nucléaire externe : figure 17A ; nombre de noyaux dans la couche nucléaire interne : figure 17B ; profondeur de la couche plexiforme externe en (µm) : figure 17C ; zone relative (%) de rétines sauvées par la greffe : figure 17D) des rétines de rats greffés avec des cellules RPE primaires ou des cellules IO/LD7/4.

Il doit être bien entendu, toutefois, que ces exemples sont donnés uniquement à titré d'illustration de l'objet de l'invention, dont ils ne constituent en aucune manière limitation.

### Exempte 1 : Méthodes utilisées pour la caractérisation des cellules immortalisées conformes à l'invention :

### a) Isolement et culture des cellules endothéliales d'aorte de rat

L'endothélium d'aorte est isolé par la méthode décrite par McGUIRE P.G. et al. (Lab. Invest., 1987, 57, 94-105).

L'aorte de rat est enlevée par dissection, coupée en petits morceaux (2-5 mm), qui sont placés sur des plaques comprenant 24 puits, recouvertes de collagène et contenant un milieu de culture de cellules endothéliales, de telle sorte que la face luminale des morceaux d'aorte soit en contact avec le collagène.

Le milieu de culture RPMI est supplémenté avec du sérum de veau foetal à 20 %, un supplément de croissance des cellules endothéliales à 7,5 µg/ml, de l'héparine à 80 µg/ml, de la glutamine 2 mM, de la vitamine C à 0,5 µg/ml, de la pénicilline à 100 U/ml et de la streptomycine à 100 µg/ml.

Après 3 jours, les explants sont retirés et les cellules adhérentes prolifèrent jusqu'à confluence. A confluence, les cellules ont une morphologie pavimenteuse, caractéristique des vaisseaux endothéliaux, expriment le facteur de Von Willebrand et prolifèrent dans un milieu contenant de la D-valine.

Les cellules sont utilisée après trois passages (stade le plus précoce pour une utilisation expérimentale).

### b) Protocole de mise en oeuvre de la microscopie électronique, pour l'étude morphologique des différentes cellules obtenues.

Les monocouches des cellules immortalisées (rétiniennes endothéliales et RPE) sont mises en culture dans des plaques contenant 24 puits, jusqu'à confluence. Les cellules sont fixées avec un mélange de paraformaldéhyde à 1 % et de glutaraldéhyde à 3 % dans du cacodylate de sodium 0,1 M-HCl (pH 7,4) ou dans du glutaraldéhyde à 2,5 % dans du cacodylate de sodium 0,1 M, tamponné à pH 6,9, par l'addition d'acide tannique à 0,5 % (p/v).

Après rinçage, 3 fois 5 minutes dans un tampon au cacodylate de sodium (pH 7,4), les cellules sont à nouveau fixées pendant 2 heures à 4°C et à l'abri de la lumière, dans une solution aqueuse à 1 % de tetroxyde d'osmium, déshydratées, en présence d'alcools à différents taux (1x10 min 50-90 %, 4x10 min 100 %), incluses dans de l'araldite et wlcanisées à 60°C pendant 12 heures. Des sections semi-fines (1 µm) et ultra-fines sont préparées (50 nm), à l'aide d'un microtome Leica Ultracut 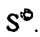 Les sections semi-fines sont colorées pour être observées au microscope, avec du bleu de toluidine à 1 % dans de l'éthanol à 50 % et les sections ultra-fines sont colorées séquentiellement avec du citrate de plomb à 1 % dans de l'éthanol à 50 % et dans du citrate de plomb et observées et photographiées avec un microscope électronique à transmission JEOL 1010, opérant à 80 kV.

### c) Protocole de détection des antigènes de surface endothéliaux et épithéliaux par ELISA, immunohistochimie et cytométrie de flux.

### * ELISA :

Les cellules endothéliales rétiniennes de rat (culture primaire et cellules immortalisées) sont ensemencés à densité de confluence sur des plaques comprenant 96 puits et qui ont été préalablement recouvertes de collagène de type IV à 0,05 %.

Avant l'étalement des cellules, le collagène est fixé dans de la vapeur d'ammoniac et les plaques sont lavées deux fois avec du HBSS. Les cellules sont cultivées pendant 3 jours avant leur utilisation expérimentale. Après les traitements expérimentaux, les cellules sont lavées 4 fois dans une solution salée et tamponnée de Hanks (HBSS) et fixées avec du glutaraldéhyde à 0,1 % dans une solution saline tamponnée au phosphate (PBSA) pendant 10 minutes à température ambiante.

Les cellules sont lavées avec un tampon Tris-HCl 50 mM pH 7,5 pendant 20 minutes à température ambiante. Les anticorps primaires sont dilués dans 100 µl d'HBSS contenant 100 µg/ml d'IgG normales de lapin et 4 mg/ml de sérum albumine bovine, puis incubés avec les cellules pendant 45 minutes à 37°C. Les cellules sont lavées 4 fois avec du PBSA contenant du Tween-20 à 0,2 %, puis incubées avec une IgG anti-souris biotinylée (1 :700 ; Amersham) pendant 45 minutes à 37°C. Les cellules sont à nouveau lavées 4 fois avec du PBSA contenant du Tween-20 à 0,2 % et incubées avec de la peroxydase de raifort-streptavidine (1 :700 ; Amersham) pendant 45 minutes à 37°C. Les cellules sont lavées 4 fois dans du PBSA contenant du Tween-20 à 0,2 % et incubées avec 100 µl de tétraméthylebenzidine dans un tampon citrate-acétate (pH 5) pendant 10 minutes. Les réactions sont arrêtées par l'addition de 50 µl d'acide sulfurique 1 M et le produit de la réaction est quantifié (densité optique à 450 nm).

### * Etudes histochimiques :

Les cultures primaires et les cellules immortalisées sont ensemencées sur des lames (Gibco/BRL) et cultivées à confluence.

La détection des antigènes de surface est effectuée en lavant les cellules dans de l'HBSS puis en bloquant à l'aide d'HBSS contenant 100 µg/ml d'IgG normale de lapin et 4 mg/ml de sérum albumine bovine.

Les anticorps primaires sont ensuite ajoutés aux cellules non-fixées et incubées pendant 1 heure sur de la glace, lavées et un deuxième anticorps biotinylé spécifique est ajouté et incubé pendant 30 minutes. Après lavage, la streptavidine marquée au FITC est incubée avec les cellules pendant 15 minutes. Les cellules sont alors fixées, montées et observées sur un Axiophot Zeiss. Pour les antigènes intracellulaires, les cellules sont fixées et perméabilisées comme décrit pour la méthode ELISA

### * Cytométrie de flux :

La cytométrie de flux des cultures rétinales confluentes est réalisée sur un appareil FACScan (Becton-Dickinson). Après lavage, les monocouches de cellules sont dissociées dans I'HBSS contenant 1 mg/ml de collagénase/dispase et de l'EDTA à 0,2 % et les cellules sont remises en suspension dans un tampon PBS.

5.10⁴ cellules/flacon sont incubées pendant 1 heure avec l'anticorps primaire sur de la glace, suivie par une deuxième incubation d'l heure avec un anticorps F(ab')₂ de lapin anti IgG de souris conjugué au FITC, en présence de sérum de rat normal à 20 %. Après 2 lavages, les cellules sont remises en suspension dans du tampon PBS et analysées. Les cellules non-colorées sont utilisées pour la calibration et les cellules colorées uniquement avec le deuxième anticorps sont utilisées pour établir le bruit de fond.

### d) Protocole pour l'étude de la migration des lymphocytes T à travers la monocouche :

La capacité des cellules immortalisées à permettre la migration trans-endothéliale des cellules T spécifiques de l'antigène est déterminée comme décrit dans GREENWOOD J. et al. (Immunol., 1993, 80, 401-406).

Les cellules T sont ajoutées (2.10⁵ cellules/ml/puits) dans des plaques 24 puits contenant des monocouches de culture cellulaire primaire ou immortalisées endothéliales rétiniennes et RPE.

Les cellules T sédimentent et migrent en 4 heures. Pour évaluer le taux de migration, les co-cultures sont placées dans un microscope à contraste de phase, maintenues à 37°C et à un taux de CO₂ de 5 %.

Un champ de 200x200 µm est choisi de manière aléatoire et enregistré pendant 10 minutes, reparties sur la période de 4 heures, à l'aide d'une caméra. Les données sont exprimées comme le pourcentage de lymphocytes totaux dans un champ qui ont migré à travers la monocouche ; un minimum de 6 puits/essai est analysé.

### Exemple 2 : Préparation d'une lignée conforme à l'invention : cellules endothéliales rétiniennes de rat.

### a) Isolement et culture des cellules endothéliales rétiniennes

Des cellules endothéliales sont dérivées de rats Lewis femelles âgées de 4 à 6 semaines et dépourvues d'agents pathogènes. Les cellules rétiniennes sont isolées et cultivées selon les méthodes décrites par GREENWOOD J. (J. Neuroimmunol., 1992, **39,** 123-132) et ABBOTT N.J. et al. (Cell. Sci., 1992, **103,** 23-37). Ces techniques produisent des cultures primaires dont la pureté est supérieure à 95 %. Les rétines de rat sont dispersées par digestion enzymatique, les fragments de microvaisseaux sont séparés des cellules seules par centrifugation, qui sont lavées et mises en culture dans des flacons recouverts de collagène. Le milieu de croissance consiste en milieu Ham's F-10 (Sigma) supplémenté avec du sérum à 17,5 % (Advanced Protein Products Ltd), un supplément de croissance de cellules endothéliales à 7,5 µg/ml (Advanced Protein Products Ltd), de l'héparine à 80 µg/ml, de la glutamine 2 mM, de la vitamine C à 0,5 µg/l, de la pénicilline à 100 U/ml et de la streptomycine à 100 µg/ml.

Les cultures sont maintenues à 37°C et dans du CO₂ à 5 % ; le milieu est remplacé tous les trois jours jusqu'à confluence.

### b) Immortalisation des cellules

Un vecteur rétroviral contenant le gène grand T SV40, déficient pour la réplication, obtenu conformément à la technique décrite par P.S. JAT et al. (Mol. Cell. Biol., 1986, 1204-1217) est produit en quantité dans des lignées de fibroblastes sélectionnées (lignée SVU19.5).

Ce vecteur rétroviral code en particulier pour un antigène grand T tsa58, sensible à la température, associé au gène codant pour la néomycine, en tant que marqueur de sélection. Il est obtenu à partir du surnageant de culture des cellules SVU19.5, après passage à travers un filtre 0,45 µm, pour éliminer les cellules productrices. Il est ajouté à une culture primaire de cellules endothéliales, telles que préparées en a) (transfection).

200 µl de virus dans 2 ml d'un milieu contenant du polybrène à 8 µg/ml (Aldrich) sont ajoutés aux cellules endothéliales ; le tout est incubé pendant 4 heures à 37°C, dans un incubateur sous atmosphère CO_{2,} en remuant le flacon toutes les 15 minutes.

Après l'incubation, le milieu est éliminé et 5 ml de milieu frais sont ajoutés ; le tout est remis en culture une nuit. Les cellules sont ensuite maintenues dans l'incubateur pendant 48 heures. Les cellules sont alors étalées sur un milieu sélectif contenant de la généticine à 200 µg/ml (G418, Gibco) et les lignées parentales immortalisées sont obtenues par sélection des colonies résistantes. Après clonage par dilution limite de la lignée parentale on obtient en particulier le clone IO/JG2/1, sélectionné, pour une étude plus approfondie.

### c) caractéristiques du clone IO/JG2/1

Ce clone est cultivé jusqu'au trentième passage, sans différences morphologiques ou phénotypiques significatives.

### • Morphologie

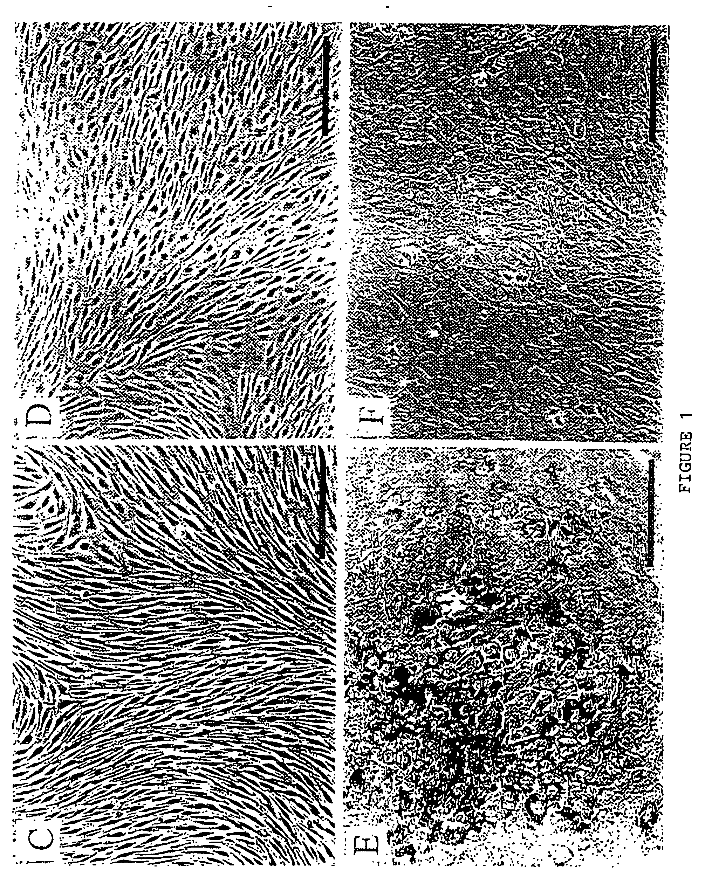

Les cultures primaires de cellules endothéliales rétiniennes présentent une morphologie fusiforme, caractéristique de ces cellules (Figure 1C). Le clone IO/JG2/1 immortalisé conserve cette morphologie caractéristique (figure 1D).

### • Apparence ultrastructurale

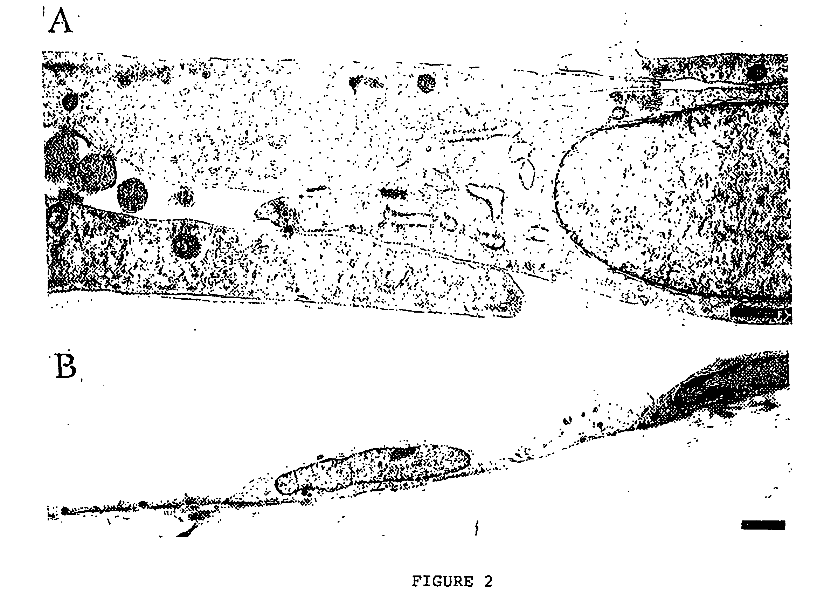

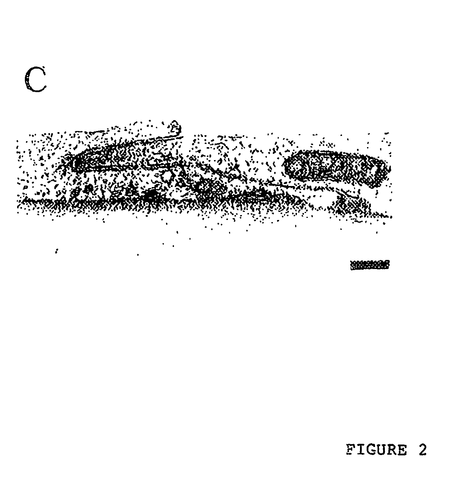

L'apparence ultrastructurale du clone IO/JG2/1 (figure 2 : B, C) est similaire à celle des cultures primaires. On observe un noyau volumineux avec la présence d'hétérochromatine périphérique ainsi que de nombreux organites cytosoliques, tels que mitochondries, réticulum endoplasmique et polysomes. Les jonctions entre les cellules présentent souvent une interdigitation avec des régions de densité cytoplasmique aux points de contact étroits. Les cellules reposent sur une lame basale.

### • Expression de l'antigène grand T tsa58

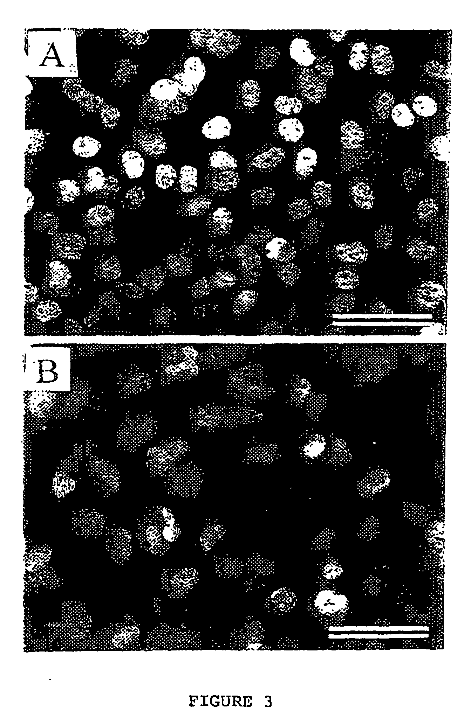

Toutes les cellules endothéliales immortalisées rétiniennes sélectionnées dans le milieu G418 montrent une coloration nucléaire importante avec des anticorps dirigés contre l'antigène grand T (figure 3A : IO/JG2/1 ; figure 3B : IO/LD7/4), alors qu'aucune coloration n'est observée dans les cellules endothéliales des cultures primaires.

### • Expression de marqueurs endothéliaux

Le clone IO/JG2/1 exprime un certain nombre d'antigènes spécifiques des cellules endothéliales ; les résultats obtenus sont illustrés au Tableau I.

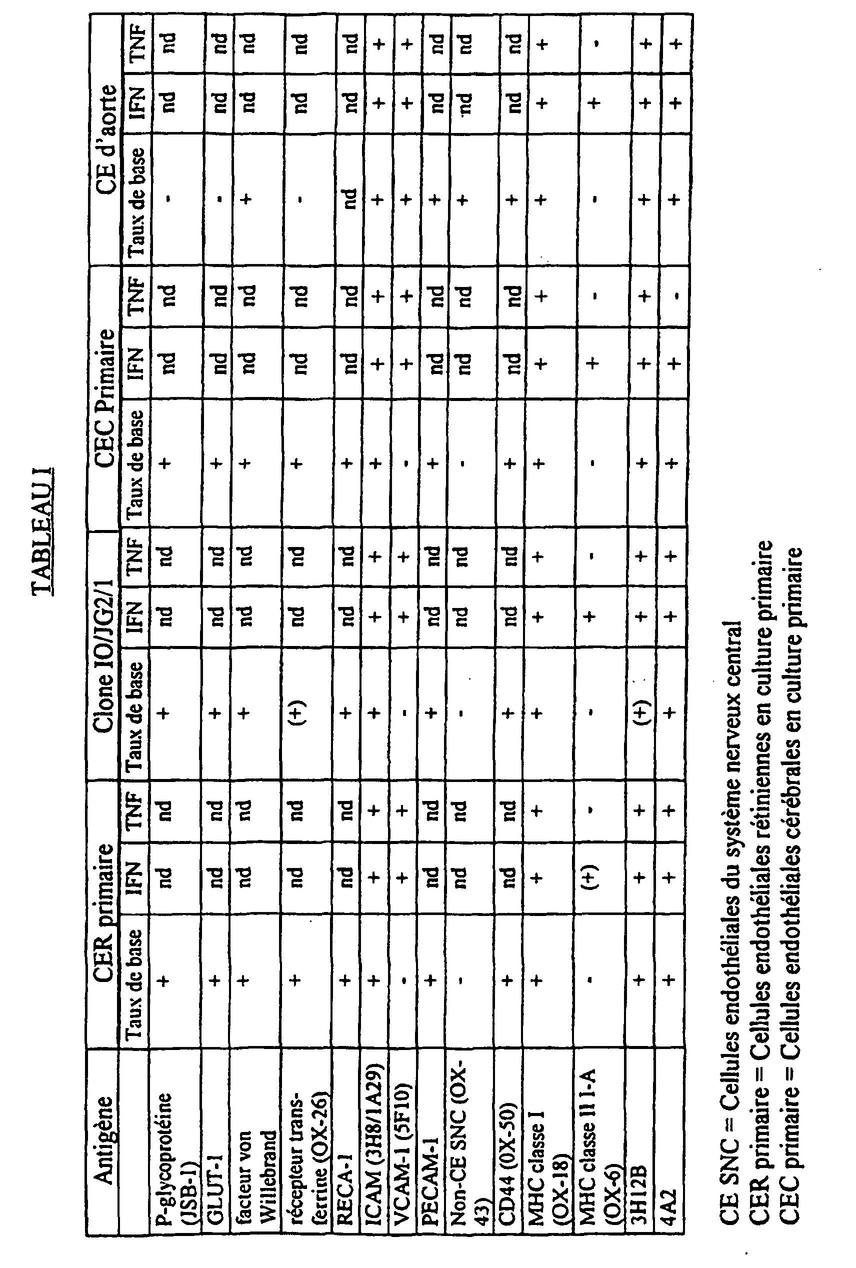

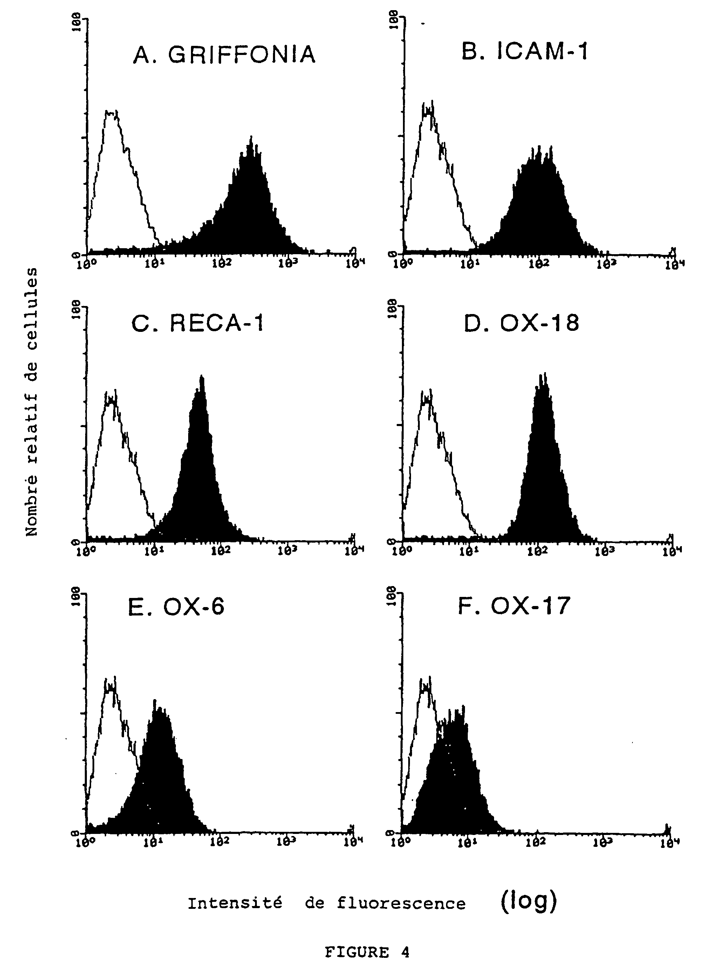

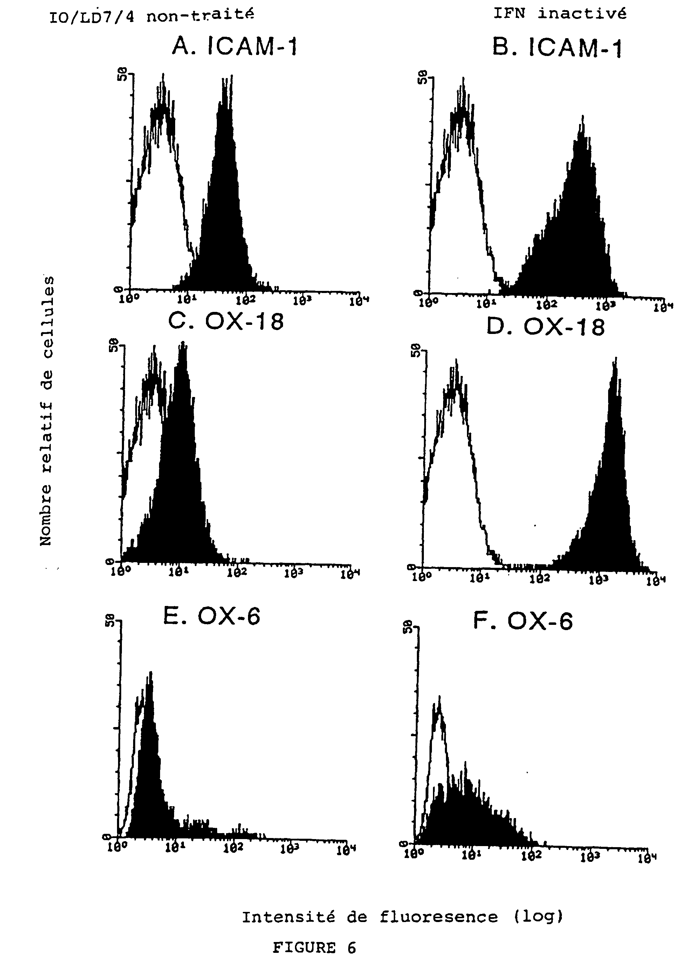

Le Tableau I montre en particulier que ce clone exprime notamment le facteur de von Willebrand, l'antigène RECA-1, l'antigène ICAM-1, dont l'expression peut également être induite par traitement avec 100 U/ml d'IFN-γ ou de TNFa pendant 24 heures (Tableau I et figures 4 et 6) et l'antigène VCAM-1, après induction par les cytokines précitées (200 U/ml d'IFN-γ ou de TNF pendant 24 heures ou 48 heures) (voir Tableau I).

### • Expression de marqueurs endothéliaux spécifiques du SNC

Le Tableau I montre également que le clone IO/JG2/1 exprime de manière constitutive un certain nombre de marqueurs spécifiques des cellules endothéliales du SNC, notamment la P-glycoprotéine, GLUT-1, et le récepteur de la transferrine (voir Tableau I) ; toutefois, le clone IO/JG2/1 n'exprime pas certains des antigènes spécifiques des cellules endothéliales cérébrales et notamment les antigènes 1A8B et 2A4, ce qui permet de le différencier de l'endothélium cérébral (Tableau II ci-après).

### • Comparaison de l'expression des antigènes endothéliaux sur les cultures primaires et dans les lignées immortalisées et par rapport aux cellules endothéliales périphériques.

Comme précisé ci-dessus les cultures primaires d'endothélium rétinien et les clones dérivés exprimant l'antigène grand T présentent une expression constitutive des marqueurs spécifiques des cellules endothéliales du SNC, à savoir la P-glycoprotéine, GLUT-1 et le récepteur de la transferrine (Tableau I), alors que l'endothélium aortique n'exprime pas ces antigènes mais exprime l'antigène OX-43, considéré comme spécifique des cellules endothéliales périphériques ; l'antigène OX-43 n'est effectivement pas exprimé, ni par les cultures primaires, ni par les cultures immortalisées de cellules endothéliales cérébrales et rétiniennes (Tableau I).

Ces différentes cultures ont en outre été criblées par rapport à un panel d'antigènes considérés comme spécifiques des cellules endothéliales cérébrales. Les résultats sont illustrés au Tableau II.

**TABLEAU II**

| Antigène | CER primaire | Clone IO/JG2/1 | CEC Primaire | CE d'aorte |
|---|---|---|---|---|
| 3B7 | + | - | + | + |
| 3D11 | + | + | + | + |
| 3D7B | + | (+) | + | + |
| 4C6C | + | (+) | + | + |
| 2F1B | + | + | + | + |
| 2A4 | - | - | + | + |
| 4E3 | + | + | + | + |
| 2A5 | + | + | + | + |
| 1A8B | - | - | + | + |
| 1C1 | + | - | + | + |
| 1C11 1 | + | + | + | + |
| 1D2 | + | - | + | + |
| 4E8.C4 | + | + | + | + |

### • Expression des antigènes du système majeur d'histocompatibilité

Toutes les cultures endothéliales présentent une expression constitutive des antigènes majeurs d'histocompatibilité de classe I (OX-18, voir Tableau I et figures 4, 6 et 7), qui est induite par 100 U/ml d'IFN-γ recombinant de rat, pendant 24 heures.

Les cultures primaires de cellules endothéliales rétiniennes et cérébrales aussi bien que les lignées parentales et les clones exprimant l'antigène grand T ne présentent pas ou très peu d'expression des antigènes majeurs d'histocompatibilité de classe II.

Des cultures de cellules endothéliales traitées pendant 24 heures avec 100 U/ml d'IFN-γ recombinant uniquement présentent une induction importante de certains antigènes de classe II : OX-6 et OX-17 (voir Tableau I et figure 4).

### • Migration des lymphocytes T à travers la monocouche

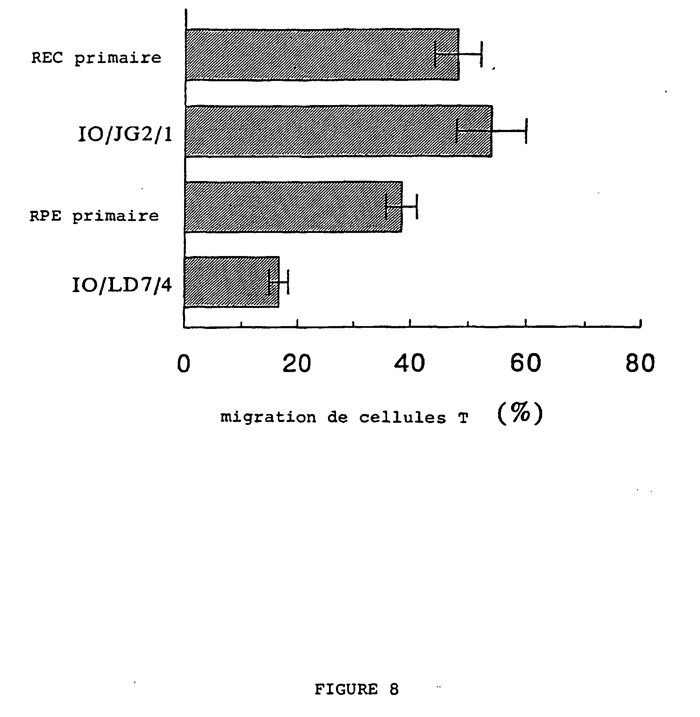

Il n'existe pas de différence significative dans la capacité des cellules primaires et des cellules immortalisées à supporter la migration T spécifique. Le degré de migration à travers les monocouches au cours d'un essai d'une durée de 4 heures est de 52±5 % pour les cellules endothéliales primaires cérébrales, de 48±4 % pour les cellules endothéliales primaires rétiniennes et de 54±6 % pour le clone endothélial rétinien IO/JG2/1 (figure 8).

### Exempte 3 : Préparation d'une lignée conforme à l'invention : cellules épithéliales rétiniennes de rat.

### a) Isolement et culture des cellules épithéliales pigmentaires rétiniennes

Les cellules épithéliales pigmentaires rétiniennes de rat sont isolées à partir de rats PVG, âgés de 6 à 8 jours, conformément à la méthode de CHANG et al. (Curr. Eye Res., 1991, 10, 1081-1086). Les yeux sont prélevés et les globes intacts sont digérés avec de la dispase à 2 %, pendant 30 minutes. Les yeux sont ensuite disséqués, de manière à retirer la cornée et le corps vitré ; la rétine est ensuite isolée et incubée 15 minutes dans un milieu de culture. Après incubation, des couches de cellules rétiniennes pigmentaires épithéliales (cellules RPE) sont séparées de la neurorétine et trypsinisées, de manière à obtenir une suspension cellulaire. Les cellules sont étalées dans des flacons de cultures de tissus et cultivées jusqu'à semi-confluence. Le milieu de culture est constitué d'un milieu Ham's F-10, supplémenté avec du sérum de veau foetal à 20 %, de l'HEPES 20 mM, du bicarbonate de sodium à 7,5 %, de la glutamine 2 mM, de la pénicilline à 100 U/ml et de la streptomycine à 100 µg/ml. Ces cultures primaires croissent sous la forme de monocouches pigmentées, positives pour les cytokératines et l'épitope spécifiques des RPE, le RET-PE2 (NEILL et al., Exp. Eye Res., 1990, 51, 573-583).

### b) Immortalisation des cellules

Elles sont réalisées dans les mêmes conditions que celles exposées à l'exemple 2, à l'exception du temps d'incubation avec le vecteur rétroviral, qui est de 2 heures. Les lignées parentales immortalisées sont obtenues par sélection des colonies résistantes. Après clonage par dilution limite de la lignée parentale, on obtient en particulier le clone IO/LD7/4, sélectionné pour une étude plus approfondie.

### c) caractéristiques du clone IO/LD7/4

Ce clone est cultivé jusqu'au 52ème passage, sans différences morphologiques ou phénotypiques significatives.

### • Morphologie

La morphologie des cellules RPE immortalisées (figure 1F) est similaire à celle des cultures primaires (figure 1E). Contrairement aux cultures primaires, les cellules immortalisées ne sont pas pigmentées.

### • Apparence ultrastructurale

Bien que les cellules immortalisées du clone IO/LD7/4 ne soient pas pigmentées, lorsqu'elles sont observées au microscope, le TEM révèle des corps denses ayant l'apparence de premelanosomes (figure 2A).

### • Expression de l'antigène grand T tsa58

Les cellules immortalisées RPE, sélectionnées à l'aide de G418 montrent une coloration nucléaire importante en présence d'anticorps dirigés contre l'antigène grand T (figure 3B), alors qu'aucune coloration n'est observée avec les cellules RPE en cultures primaires.

### • Expression de marqueurs RPE

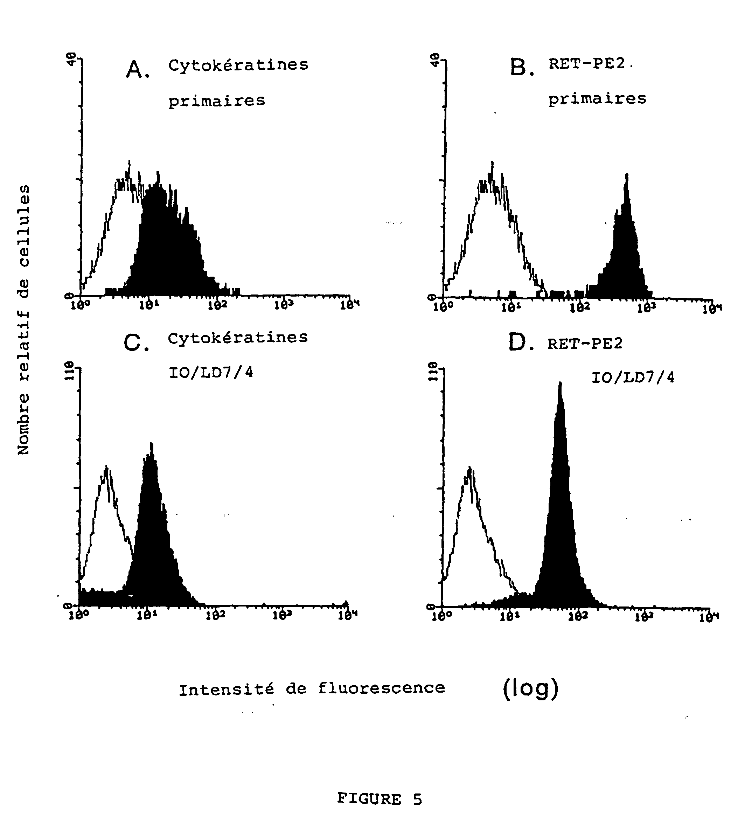

Les cultures primaires de RPE et le clone immortalisé IO/LD7/4 expriment l'antigène spécifique des cellules RPE, à savoir RET-PE2 (figure 5). De plus, l'expression des cytokératines, qui sont habituellement utilisées pour identifier des cellules RPE, sont présentes, au même degré, aussi bien dans les cultures primaires que dans les cellules immortalisées, comme cela est illustré dans l'analyse en cytométrie de flux (figure 5).

### • Expression des antigènes du système majeur d'histocompatibilité

Toutes les cultures épithéliales pigmentaires présentent une expression constitutive des antigènes majeurs d'histocompatibilité de classe I (OX-18, voir Tableau I et figures 4, 6 et 7), qui est induite par 100 U/ml d'IFN-γ recombinant de rat, pendant 24 heures.

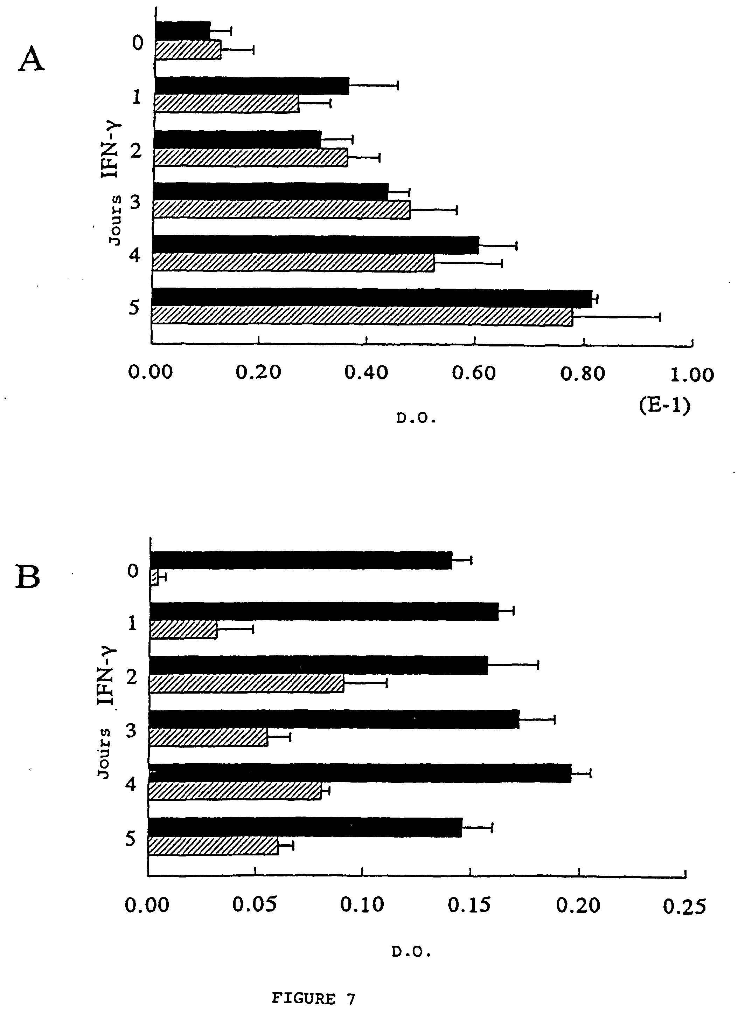

A la fois les cultures primaires et les cellules RPE immortalisées sont incapables d'exprimer les antigènes du complexe majeur d'histocompatibilité de classe II I-A ou de classe II 1-E ; toutefois, après 5 jours d'activation, il existe une expression faible mais significative à la fois de I-A (figures 6 et 7) et de I-E.

### • Expression des molécules d'adhésion

Les cultures cellulaires RPE primaires et immortalisées n'expriment pas de manière constitutive le VCAM-1, mais après 3-5 jours d'activation avec de l'IFN-γ, on observe des taux faibles d'expression (figure 7).

### • Migration des lymphocytes T à travers la monocouche

La migration à travers les monocouches de cellules RPE primaires et immortalisées diffère de manière significative : les cellules primaires présentent un degré de migration significativement supérieur (38±3 %) à celui des cellules immortalisées IO/LD7/4 (17±2 %, p<0,01) (figure 8).

### d) Phagocytose de segments externes des bâtonnets par les cellules IO/LD7/4 in vitro

### • Méthode :

Des cellules IO/LD7/4 sont cultivées à confluence sur des lamelles Thermanox® dans 4 puits de plaques de culture de tissus. Le milieu est aspiré et remplacé par un milieu contenant une suspension de cellules rétiniennes adultes dissociées. 24 heures plus tard, la suspension rétinienne est éliminée, puis les lamelles sont rincées et traitées pour une observation au microscope électronique. Les cellules sont également cultivées sur des lamelles recouvertes de Matrigel®, rincées, fixées et colorées au crésyl-violet.

### • Résultats :

La présence de matériel rétinien (segments externes de photorécepteurs) dissocié dans les phagosomes peut être distinguée dans les micrographies électroniques de cellules IO/LD7/4.

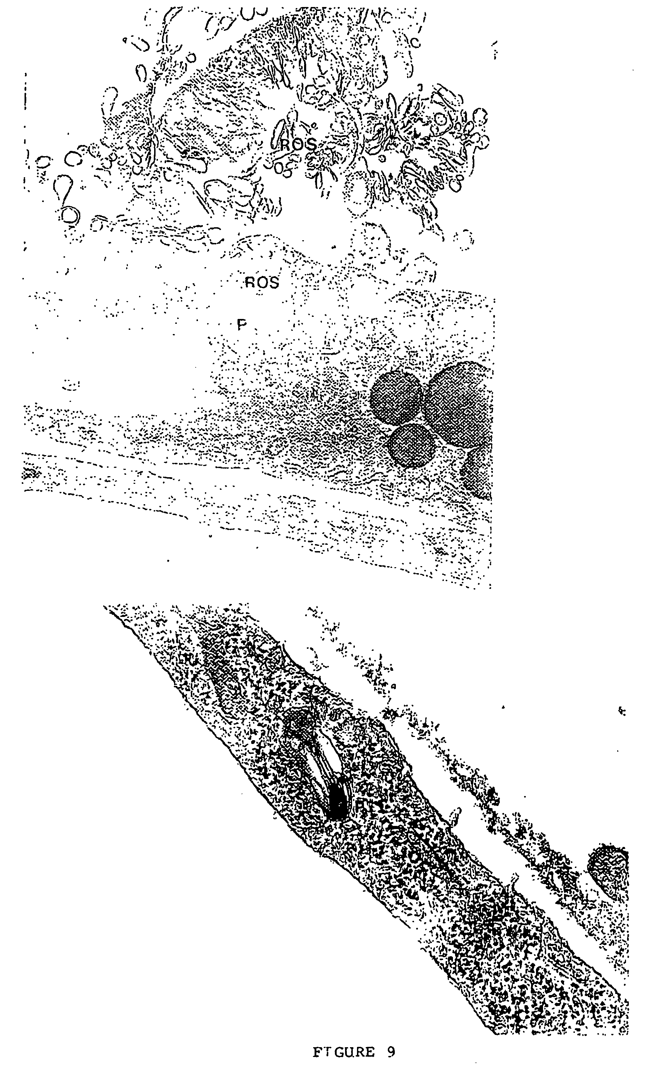

Des segments externes peuvent être identifiés à la fois, en suspension au dessus des cellules RPE primaires et dans les phagosomes. Une fine couche de matériel dense électroniquement est observable sous la couche de cellules en culture et est interprétée comme correspondant à une membrane basale produite par les cellules (figure 9).

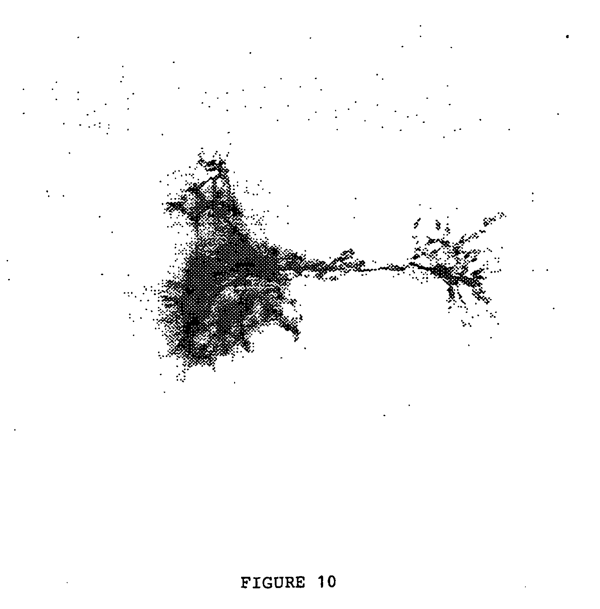

Les cellules cultivées sur Matrigel® entraînent une contraction de la matrice (figure 10).

### EXEMPLE 4 : Implantation dans l'espace sous-rétinien de cellules IO/LD7/4.

Les cellules RPE immortalisées selon l'invention sont implantables dans l'espace sous-rétinien et permettent de sauver les photorécepteurs de la dégénération et constituent une source de production de cellules donneuses particulièrement avantageuse.

### 1. Expérience pilote : greffe de cellules IO/LD7/4 à des rats Sprague-Dawley et à des rats RCS.

### • Méthodes

Des cellules immortalisées selon l'invention (cellules IO/LD7/4) sont injectées dans l'espace sous-rétinien de 8 rats Sprague-Dawley anesthésiés, âgés de 12 semaines et de 6 rats RCS, âgés de 4 semaines.

L'oeil est soumis à une rotation vers le nez et ancré ; une incision est effectuée avec un scalpel très fin (couteau microchirurgical ayant un angle de 15°) à travers les couches scléreuse et choroïdale, pour faciliter l'insertion d'une micropipette. Les cellules (2.10⁴/µl) sont injectées dans l'espace sous-rétinien à l'aide d'une micropipette qui est attachée à une seringue Hamilton de 10 µl.

La moitié du groupe est traité avec de la cyclosporine par voie orale (2,1 mg/rat/ jour), pendant toute la durée de l'expérience.

Les rats de chaque groupe sont anesthésiés avec une dose létale d'anesthésique (Euthatal®) puis une perfusion intracardiaque successivement avec du PBS et un agent fixateur des tissus est réalisée ; les animaux sont alors énucléés.

Les yeux sont cryoprotégés et inclus dans de l'OCT® (Miles) (agent de cryoprotection).

Des coupes en série sont préparées (épaisseur de 14 µm) et colorées au crésyl-violet et avec un anticorps monoclonal dirigé contre l'antigène SV40-T et contre le PCNA (antigène nucléaire de cellule proliférative).

### • Résultats

On n'observe aucune formation de tumeur dans les yeux d'animaux ayant reçu des greffes de cellules IO/LD7/4.

On n'observe aucune réponse immunitaire dans les yeux d'animaux qui n'ont pas reçu un traitement immunosuppresseur.

La couche cellulaire épithéliale, dans la plupart des coupes, est simple (monocouche) ; toutefois, dans certaines régions, une multicouche est observée.

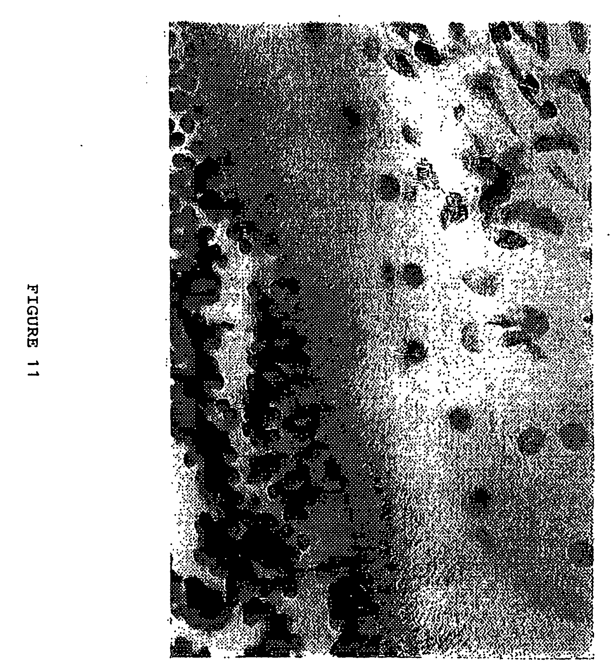

Lorsque les blocs ont été sectionnés « en face », les cellules immortalisées selon l'invention, *in vivo*, présentent les caractéristiques phénotypiques hexagonales des cellules RPE primaires, même si ces caractéristiques sont perdues *in vitro* (figure 11) ; cela est évident lorsque le transplant présente plus d'une couche cellulaire.

La protection des photorécepteurs est observée dans toutes les rétines de rats RCS qui présentent des greffes de cellules IO/LD7/4. Toutes les coupes colorées avec les anticorps SV40-T et PCNA sont négatives.

### 2. Comparaison entre des greffes de cellules RPE primaires fraîchement récoltées et des greffes de cellules IO/LD7/4 sur la fonction visuelle.

### • Méthodes

11 rats dystrophiques RCS (3-4 semaines) sont greffés soit avec des cellules RPE primaires, soit avec des cellules IO/LD7/4, par injection dans l'espace sous-rétinien de chaque oeil, comme décrit en 1.

Un groupe séparé d'animaux est soumis à une injection de milieu uniquement (opération contrôle).

### a) Evaluation du réflexe pupillaire à la lumière (RPL)

Le RPL est enregistré 6 mois après la transplantation à l'aide d'un pupillomètre ; les animaux sont testés sous anesthésie (halothane/oxyde d'azote).

Le stimulus lumineux est présenté pendant 3 secondes ; les données sont collectées à l'aide du pupillomètre ISCAN et on enregistre la latence et l'amplitude de la réponse.

### • Résultats

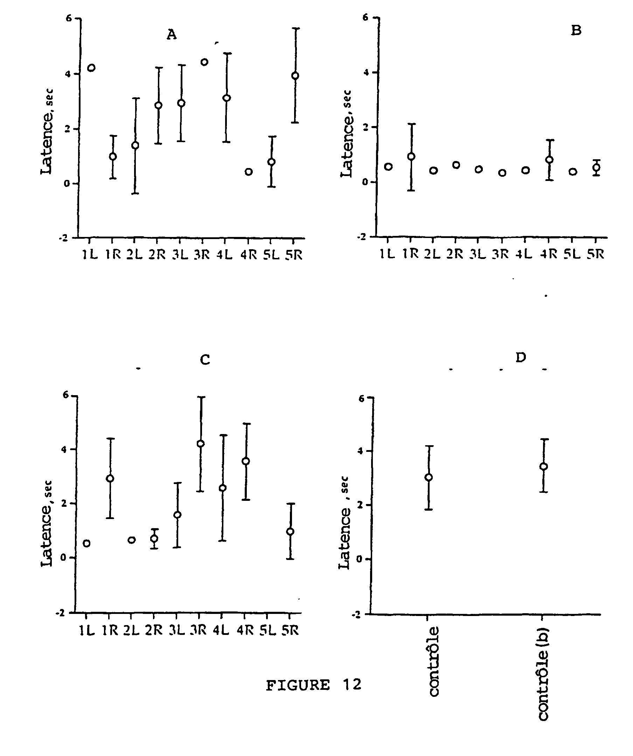

Le temps de latence des réponses RPL des animaux qui ont reçu une greffe de cellules IO/LD7/4 est significativement plus court que celui des animaux ayant reçu une greffe de cellules RPE primaires ou une injection de milieu (figure 12).

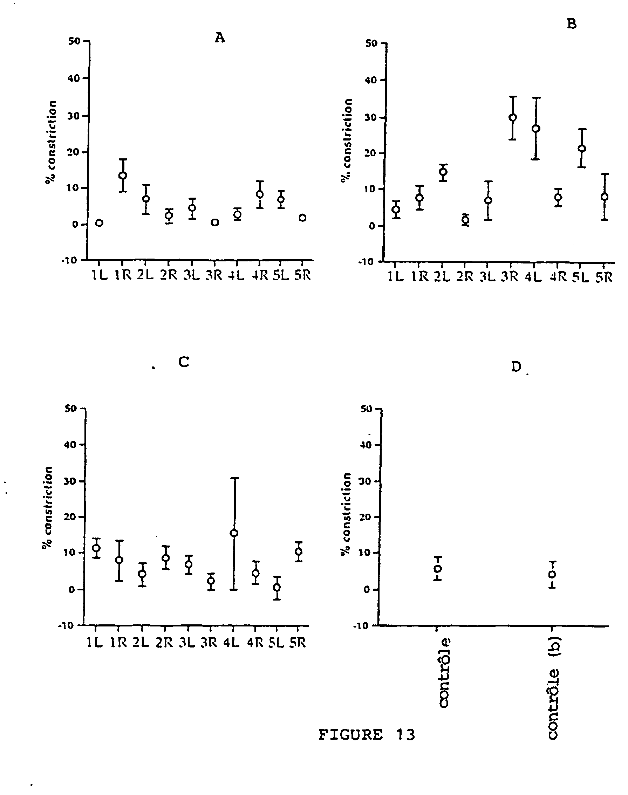

L'amplitude des réponses présente de grandes variations dans les différents groupes ; toutefois, un sous-ensemble d'animaux ayant reçu des cellules IO/LD7/4 montre une plus grande amplitude de réponse que dans les autres groupes (figure 13).

### b) Evaluation comportemental de l'acuité visuelle

Pour évaluer la capacité des rats à détecter des images visuelles, les rats sont placés dans une grande cage, dont les parois peuvent être changées (parois unies ou parois décorées).

2 rats sont présents dans chaque cage, pendant le test ; leur activité est mesurée pendant 5 minutes.

Dans un premier temps, l'animal explore son environnement mais s'y habitue.

Dans un deuxième temps, l'activité de l'animal augmente, seulement si les parois sont changées ; en effet, si l'environnement visuel est le même, l'activité n'est pas modifiée.

L'étendue de l'activité de l'animal peut en conséquence être utilisée comme un index de sa détection et de son acuité visuelles.

### • Résultats

La capacité des rats dystrophiques à détecter les variations de leur environnement visuel diminue sur une période de 3 mois.

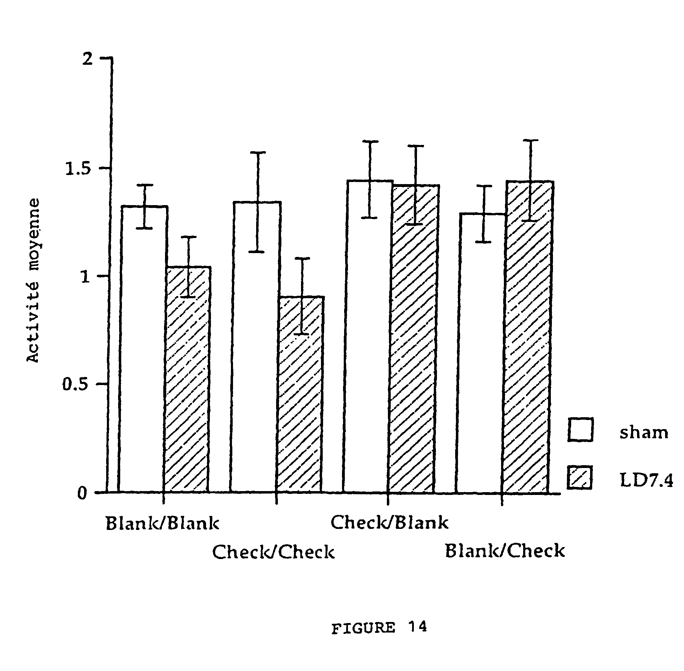

L'activité exploratoire des rats greffés avec des cellules IO/LD7/4 est importante, lors de la deuxième partie du test, c'est-à-dire lorsque l'environnement a été modifié (passage des parois unies aux parois décorées ou vice-versa) (figure 14).

### c) Estimation électrophysiologique

La tête et les yeux des rats anesthésiés sont immobilisés par un appareil de stabilisation et des sutures, respectivement. Le collicule supérieur contralatéral à l'oeil stimulé est exposé. Les animaux sont adaptés à un niveau lumineux de 0,34 cd/m² pendant 1 heure, avant l'enregistrement, pour permettre l'évaluation simultanée des cônes et des bâtonnets. Le rat est orienté face à un hémisphère translucide (rayon de 55 cm), de telle manière que l'oeil testé soit au centre. Les stimuli, comprenant une lumière fixe de 10° de diamètre et de 5,8 cd/m² d'intensité, sont projetés sur la surface de l'hémisphère.

Différents champs réceptifs (multi-unitaires ou uniques) sont enregistrés à partir de la couche la plus superficielle du collicule supérieur (CS) (environ 200 µm de la surface du CS), en utilisant des électrodes en fibre de carbone, recouvertes de verre.

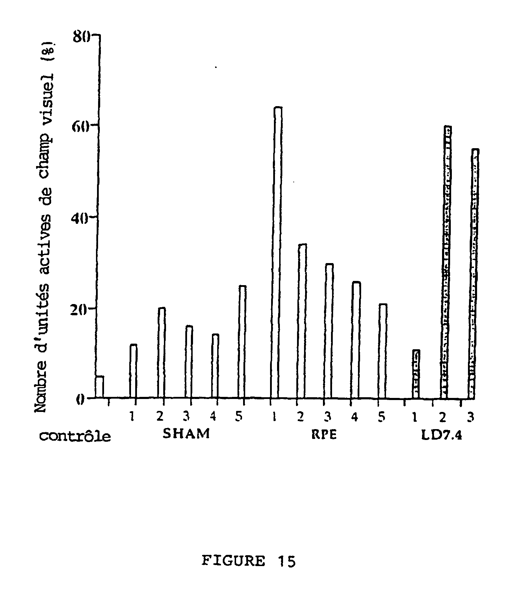

Les enregistrements prennent en compte l'ensemble du CS sur la base d'un système de grille à pas de 200 µm (figures 15 et 16).

### • Résultats

Les axones de la rétine se projettent sur le CS d'une manière précise et bien définie ; en conséquence, des modifications dans les enregistrements obtenus à partir du CS reflètent bien les changements intervenus dans la rétine (figure 15).

Un scotome de petite dimension commence à se développer à 6 semaines, chez les rats dystrophiques ; il occupe la moitié du champ visuel à 3 mois et inclut l'ensemble de la rétine à 6 mois.

Les greffes RPE primaires et IO/LD7/4 ralentissent cette détérioration du champ visuel.

Les greffes de cellules IO/LD7/4 apparaissent plus efficaces.

### d) Evaluation morphologique de la prévention de la perte des photorécepteurs après greffe de cellules RPE primaires

4 yeux ayant reçu soit des cellules RPE primaires, soit des cellules IO/LD7/4 ont été examinés.

En utilisant un logiciel d'analyse d'image associé à un microscope (DMR Leica), l'épaisseur de chacune des couches cellulaires de la rétine est mesurée sur les rats dystrophiques âgés de 6 mois et le nombre de noyaux des couches nucléaires internes et externes est compté.

La proportion de rétine sauvée est estimée.

### • Résultats

A 6 mois, on n'observe aucune couche de photorécepteurs chez les rats dystrophiques. II existe quelques cellules dans la couche nucléaire interne qui peuvent être considérées comme des vestiges de photorécepteurs.

Les greffes de cellules immortalisées montrent des régions importantes de cellules sauvées chez des animaux de 6 mois.

La proportion de zone rétinienne sauvée par les greffes de cellules RPE primaires est de 6 % et de 13,8 % avec une couche nucléaire externe (CNE) d'une épaisseur égale à 3 étages de noyaux, alors que le pourcentage de rétine sauvée par les greffes de cellules IO/LD7/4 est de 27,9 % et de 36,3 % avec une CNE d'une épaisseur égale à 5 étages de noyaux.

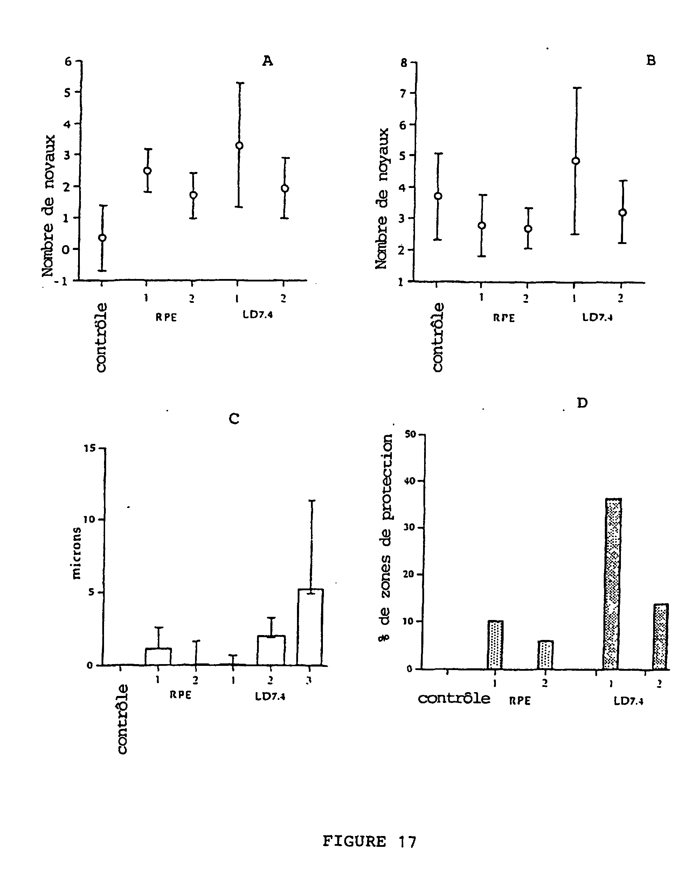

Une différence importante entre ces coupes est la présence d'une couche plexiforme externe distincte dans les yeux greffés avec des cellules IO/LD7/4 (figure 17).

### 3. Migration des cellules IOILD7/4 in vivo

### • Méthode

15 rats dystrophiques, âgés de 4 semaines sont greffés avec des cellules IO/LD7/4 fluorescentes.

10 des rats receveurs sont greffés sur les 2 yeux ; les 5 autres rats sont greffés uniquement sur un oeil.

Les animaux (2 transplantés sur les 2 yeux et un transplanté uniquement sur un oeil) sont tués 3, 7, 14, 28, 42 et 98 jours après la greffe, par administration d'une dose létale d'anesthésique (perfusion intracardiaque en présence de PBS).

Les animaux sont énucléés et les yeux sont fixés dans du paraformaldéhyde à 4 % pendant 6 heures.

Le tissu est cryoprotégé et inclut dans de l'OTC.

Des coupes sont préparées (épaisseur de 14 µm) et 3 séries sont constituées.

La série A est colorée au crésyl-violet ; la série B est colorée avec un anticorps anti-microglie ; les séries sont examinées en utilisant un microscope à fluorescence.

### • Résultats

Les cellules marquées sont localisées dans tous les yeux greffés jusqu'à 14 jours après l'opération mais sont plus difficilement identifiables plus tard, en raison de l'élimination du marqueur.

Lorsque la coloration est bien visible, les cellules marquées occupent jusqu'à 30 % de la rétine.

La coloration au crésyl-violet des coupes adjacentes confirme l'action des greffes sur le sauvetage des photorécepteurs chez tous les transplants.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

MICRO-ORGANISMES
Feuille facultative relative au micro-organisme mentionné en page 4 ligne 15 de la description ¹
A. IDENTIFICATION DU DÉPOT ¹
D'autres dépôts sont identifiés sur une feuille supplémentaire ² [ ]
Nom de l'institution de dépôt ⁴
Collection Nationale de Cultures de Micro-organismes
Adresse de l'institution de dépôt (y compris le code postal et le pays) ⁴
28 rue du Docteur Roux, 75724 PARIS CEDEX 15
Date du dépôt ⁴ 18 avril 1996 | N° d'ordre ⁴ I-1694
B. INDICATIONS SUPPLÉMENTAIRES ¹ (à ne remplir que si nécessaire). Une feuille séparée est jointe pour la suite de ces renseignements [ ]
"En ce qui concerne les désignations dans lesquelles un
brevet européen est demandé, un échantillon du micro-
organisme déposé ne sera accessible, jusqu'à la publication
de la mention de la délivrance du brevet européen ou
jusqu'à la date à laquelle la demande sera rejetée, retirée
ou réputée retirée, que par la remise d'un échantillon à un
expert désigné par le requérant. (règle 28.4) de la CBE)".
C. ÉTATS DÉSIGNÉS POUR LESQUELS LES INDICATIONS SONT DONNÉES ¹ (si les indications ne sont pas données pour tous les États désignés)
EUROPE NOUVELLE-ZELANDE
AUSTRALIE ETATS-UNIS D'AMERIQUE
CANADA
JAPON
D. INDICATIONS FOURNIES SÉPARÉMENT ¹ (à ne remplir que si nécessaire)
Les indications énumérées ci-après seront soumises ultérieurement au Bureau international ¹ (spécifier la nature générale des indications p. ex., « No d'ordre du dépôt »)
E. [ ] La présente feuille a été reçue avec la demande internationale lorsque celle-ci a été déposée (à vérifier par l'office récepteur)
(Fonctionnaire autorisé)
[ ] Date de réception (en provenance du déposant) par le Bureau international ¹⁰ C. BEDNARICK
(Fonctionnaire autorisé)
(Janvier 1985)
Formulaire PCT/RO/134 (Janvier 1981)

## Revendications

1. Lignées immortalisées de cellules de mammifères, dérivées de cellules primaires épithéliales de rétine, dénommées IO/LD7/4, **caractérisées en ce qu'**elles ont été déposées sous le n° 1-1694 en date du 18 avril 1996 auprès de la Collection Nationale de Cultures de Micro-organismes tenue par l'Institut Pasteur.

2. Lignées cellulaires vectrices, **caractérisées en ce qu'**elles comprennent au moins une lignée cellulaire selon la revendication 1, associée à un vecteur d'expression comprenant une séquence codant pour un polypeptide, une protéine ou un vecteur viral, éventuellement associé à au moins un gène de sélection et éventuellement au moins un gène marqueur et **en ce qu'**elles sont capables *in vivo* de s'intégrer à la rétine et notamment à l'espace sous-rétinien d'un mammifère hôte, à prévenir la perte des photorécepteurs et à produire ledit peptide, ladite protéine ou ledit vecteur viral.

3. Modèle d'étude et d'identification des systèmes biochimiques et cellulaires de la barrière hémato-rétinienne, **caractérisé en ce qu'**il comprend au moins une lignée cellulaire selon l'une quelconque des revendications 1 ou 2.

4. Composition pharmaceutique **caractérisée en ce qu'**elle contient au moins une lignée cellulaire selon l'une quelconque des revendications 1 ou 2.

5. Utilisation d'une lignée cellulaire selon l'une quelconque des revendications 1 ou 2, pour l'obtention d'une composition pharmaceutique pour le traitement des troubles primaires et secondaires ophtalmologiques ou neurologiques.

## Patentansprüche

1. Immortalisierte Linien von Säugetierzellen, abgeleitet aus Primär-Epithelzellen der Retina, IO/LD7/4 genannt, **dadurch gekennzeichnet, dass** sie am 18. April 1996 am Institut Pasteur bei der Collection Nationale de Culture de Micro-organismes unter der Nr. 1-1694 hinterlegt worden sind.

2. Vektor-Zelllinien, **dadurch gekennzeichnet, dass** sie mindestens eine Zelllinie nach Anspruch 1 aufweisen, die mit einem Expressionsvektor verbunden ist, der eine für ein Polypeptid, ein Protein oder einen viralen Vektor kodierende Sequenz aufweist, eventuell mit mindestens einem Selektionsgen und eventuell mit mindestens einem Markergen verbunden, und **dadurch**, dass sie *in vivo* in der Lage sind, sich in die Retina und vor allem in den subretinalen Bereich eines Wirts-Säugetiers zu integrieren, dem Verlust von Fotorezeptoren vorzubeugen und das besagte Peptid, das besagte Protein oder den besagten viralen Vektor zu produzieren.

3. Untersuchungs- und Identifikationsmodell biochemischer und zellularer Systeme der Blut-Retina-Schranke, **dadurch gekennzeichnet, dass** es mindestens eine Zelllinie nach einem der Ansprüche 1 oder 2 umfasst.

4. Pharmazeutische Zusammensetzung, **dadurch gekennzeichnet, dass** sie mindestens eine Zelllinie nach einem der Ansprüche 1 oder 2 enthält.

5. Verwendung einer Zelllinie nach einem der Ansprüche 1 oder 2 zur Herstellung einer pharmazeutischen Zusammensetzung zur Behandlung von primären und sekundären ophtalmologischen oder neurologischen Störungen.

## Claims

1. Immortal lines of mammal cells derived from primary epithelial retina cells denoted IO/LD7/4, **characterised in that** they were deposited with the National Collection of Micro-organism cultures kept by the Institut Pasteur under No. 1-1694, April 18 1996.

2. Vector cell lines, **characterised in that** they comprise at least one cell line according to claim 1, associated with an expression vector comprising a sequence coding for a polypeptide, a protein or a viral vector, possibly associated with at least one selection gene and possibly at least one marker gene, and **in that** they are capable of integrating *in vivo* with the retina and particularly the sub-retinal space in a host mammal, preventing the loss of photoreceptors and producing the said peptide, the said protein or the said viral vector.

3. Study and identification model for biochemical and cell systems in the hemato-retinal barrier, **characterised in that** it comprises at least one cell line according to either of claims 1 or 2.

4. Pharmaceutical composition **characterised in that** it contains at least one cell line according to either of claims 1 or 2.

5. Use of a cell line obtained according to either of claims 1 or 2, to obtain a pharmaceutical composition for the treatment of primary and secondary ophthalmological or neurological disorders.
